Europäisches Patentamt

European Patent Office

Office européen des brevets

(19)

(11) Numéro de publication : **0 349 381 B1**

(12) **FASCICULE DE BREVET EUROPEEN**

(45) Date de publication du fascicule du brevet :
**15.04.92 Bulletin 92/16**

(51) Int. Cl.$^5$ : **C07C 25/18, C07C 17/12**

(21) Numéro de dépôt : **89401748.2**

(22) Date de dépôt : **21.06.89**

(54) **Procédé de bromation du biphényle par BrCl.**

(30) Priorité : **27.06.88 FR 8808583**

(43) Date de publication de la demande :
**03.01.90 Bulletin 90/01**

(45) Mention de la délivrance du brevet :
**15.04.92 Bulletin 92/16**

(84) Etats contractants désignés :
**AT BE CH DE ES FR GB GR IT LI LU NL SE**

(56) Documents cités :
**FR-A- 1 312 126**

(73) Titulaire : **RHONE-POULENC CHIMIE**
**25, quai Paul Doumer**
**F-92408 Courbevoie Cédex (FR)**

(72) Inventeur : **Nonn, Alain**
**1, rue du Rhin**
**F-68120 Pfastatt (FR)**

(74) Mandataire : **Dubruc, Philippe et al**
**RHONE-POULENC CHIMIE Service Brevets**
**Chimie 25, quai Paul-Doumer**
**F-92408 Courbevoie Cédex (FR)**

## Description

La présente invention concerne un procédé de bromation du biphényle par BrCl en vue particulièrement de préparer le bromo-4 biphényle ou le dibromo 4-4'biphényle.

Le bromo-4 biphényle est un produit susceptible de nombreuses applications.

Il peut être utilisé dans la fabrication de composés agrochimiques ou pharmaceutiques. Il peut aussi servir à la préparation de cristaux liquides.

Le dibromo-4,4' biphényle, lui, est un composé donnant accès par hydrolyse au dihydroxy-4,4' biphényle, ce dernier produit étant un monomère pour polymères thermotropes.

Il s'agit donc dans les deux cas de produits intéressants d'un point de vue industriel.

Par ailleurs, un des problèmes qui se pose dans leur préparation est celui de la regio-sélectivité puisqu'il s'agit d'obtenir des produits bromés en positions 4 ou 4-4'.

Dès lors, le besoin se fait sentir d'avoir un procédé simple c'est-à-dire permettant de travailler dans des conditions douces, sélectif et de cinétique rapide.

L'objet de la présente invention est donc la mise au point d'un tel procédé.

Dans ce but, le procédé selon l'invention de bromation du biphényle est caractérisé en ce qu'on fait réagir le biphényle avec BrCl.

Ce procédé permet d'obtenir des rendements d'au moins 60% à température ambiante et pour des temps de réaction de l'ordre de l'heure.

D'autres caractéristiques et détails de l'invention apparaîtront plus clairement à la lecture de la description et des exemples concrets mais non limitatifs qui vont suivre.

La caractéristique essentielle du procédé réside dans l'utilisation, à titre d'agent bromant de BrCl.

Le procédé de l'invention peut être mis en oeuvre selon deux variantes.

La première consiste à former BrCl préalablement à sa réaction avec le biphényle.

Dans ce cas on pourra, bien entendu, utiliser tout moyen connu pour fabriquer BrCl.

A titre d'exemple, on pourra former BrCl par barbotage de chlore dans HBr ou bien par action d'HCl sur un dérivé N-bromé tel que par exemple le N-bromo N-méthylacétamide, la N-N-dibromohydantoïne, le N-bromo succinimide en solution aqueuse ou en solution organique. Un mode de réalisation préféré pour cette première variante consiste à faire barboter le chlore dans le $Br_2$ lui-même en solution dans un solvant du type $CCl_4$, $CHCl_3$, $CH_2Cl_2$, HCl aqueux par exemple.

La seconde variante, qui est préférée, consiste à former BrCl, in situ, c'est-à-dire dans le milieu même de réaction en même temps que se déroule la réaction avec le biphényle.

Dans cette variante, on introduit généralement le brome dans le milieu réactionnel dans un premier temps. Ce n'est que dans un deuxième temps qu'on ajoute le chlore et que simultanément la cinétique de réaction est accélérée.

Il est à noter qu'il serait toutefois possible d'introduire simultanément le brome et le chlore en maintenant toujours un excès de brome par rapport au chlore.

Dans les deux cas qui viennent d'être mentionnés, il est préférable de refroidir le milieu réactionnel à une température inférieure ou égale à 5°C. Pour la seconde variante à introduction différée du brome et du chlore, ce refroidissement peut se faire après l'introduction du brome et avant celle du chlore. Dans le cas d'un refroidissement, celui-ci dure au moins une partie de la réaction, généralement pendant toute sa durée. Une fois celle-ci terminée on laisse la température remonter. Le refroidissement permet de maintenir BrCl sous forme liquide ce qui facilite la maîtrise de la réaction.

Dans tous les cas, il est nécessaire d'opérer avec au moins 0,5 équivalent de $Br_2$ par rapport au substrat dans le cas d'une monobromation et d'au moins 1 équivalent de $Br_2$ par rapport au substrat dans le cas d'une dibromation. Il est tout à fait possible d'opérer avec un excès de brome par rapport aux quantités qui viennent d'être mentionnées. Pour éviter la réaction parasite de chloration, la quantité de chlore doit toujours être inférieure ou égale à celle du brome. Cette quantité dépend également du stade de bromation souhaité.

Enfin, d'une manière générale, la réaction a lieu dans un milieu comprenant un solvant. Un grand nombre de solvants peuvent être utilisés à condition qu'il s'agisse de solvants le plus inertes possibles vis-à-vis du brome et du chlore. Le solvant peut être choisi dans le groupe comprenant les hydrocarbures aliphatiques, alicycliques, aromatiques halogénés ou non, les éthers, les solvants nitrés.

On pourra citer à titre d'exemple l'hexane, le tétrachlorure de carbone, le dichlorométhane, le trichlorométhane, le dibromoéthane, le dichlorobenzène, le nitrobenzène, l'éther isopropylique, l'oxyde d'hexyle, l'oxyde de butyle.

Des exemples vont maintenant être donnés.

2

## EXEMPLE 1

Dans un ballon de 150 ml muni d'une gaine thermométrique, d'une ampoule à brome, d'un tube plongeant pour l'arrivée du chlore, d'un agitateur mécanique à deux pales et d'un réfrigérant relié à une colonne de lavage et neutralisation d'acides, on place :
. 15,4 g de biphényle (0,1 mol)
. 50 ml de dichlorométhane
. 27,2 g de brome (0,17 mol) soit un excès de 70 % par rapport à la quantité stoëchiométriquement nécessaire pour une dibromation

On refroidit ensuite à 5°C et on introduit 0,17 mole de chlore à un débit de 2,66 1/h pendant 86 minutes en maintenant la température inférieure à 5°C.

Puis on laisse remonter la température à l'ambiante et on maintient le mélange sous agitation pendant 1,5 heure.

On neutralise l'excès de brome n'ayant pas réagi par addition d'une solution aqueuse à 10 % de sulfite de sodium.

Après ajout de dichlorométhane en quantité suffisante pour solubiliser le précipité formé au cours de la réaction, on décante, la phase organique est séchée, puis le solvant est évaporé. Le solide, obtenu est séché à l'étuve à 70°C puis analysé en chromatographie en phase gazeuse. Sa masse est de 31,2 g, et sa composition en poids est la suivante :
. 87,3 % (4,4′ - diBr) soit RT (rendement de la réaction par rapport au produits transformé) 87,3 %
. 0,7 % (4-Br) RT 0,9 %
. 0,12 % bromo 2 biphényle
. 0 % biphényle => TT (Taux de transformation) = 100%

## EXEMPLE 2

On utilise le même mode opératoire que dans l'exemple 1, mais en chargeant cette fois :
. 15,4 g de biphényle (0,1 mol)
. 50 ml de dichlorométhane
. 22,4 g de brome (0,14 mol) soit un excès de 40 % tel que défini plus haut
. 0,13 mol de chlore

Le chlore est introduit sous le même débit que précédemment mais en 66 minutes. Après cette introduction de chlore, on neutralise l'excès de brome puis on procède comme décrit dans l'exemple 1.

Le produit solide récupéré a une masse de 28,22 g et la composition suivante :
. 77,7 % (4,4′ -diBr) RT = 70,3 %
. 1,4 % (4-Br) RT = 0,17 %
. 0 % biphényle => TT = 100 %

## EXEMPLE 3 :

Dans un tricol de 250 ml à agitation magnétique et équipé comme dans l'exemple 1, on charge :
. 30,8 g (0,2 mol) de biphényle
. 100 ml de dichlorométhane

A 0°C, sous agitation, on ajoute 35,2 g (0,22 mol) de brome en 30 minutes, 15 minutes après le début de l'addition du brome, on rajoute 4,71g (0,21 mol) de chlore sur une période de 2 H 15.

30 minutes après la fin de l'addition de chlore, on neutralise l'espèce bromante par une solution de sulfite de sodium.

Résultats:

```
TT = 100 %
RR (4,4' -diBr) (Rendement de la réaction par rapport au
                 substrat engagé)
               = 74,4 %
RR (4 - Br)    =   1,5 %
```

3

EP 0 349 381 B1

EXEMPLE 4 :

Dans le même appareillage que dans l'exemple précédent, on charge :
. 30,8 g de biphényle (0,2 mol)
. 80 ml de dichlorométhane

Parallèlement, on prépare BrCl en rajoutant 16,4 g de chlore (0,23 mol) à 35,2 g de brome (0,22 mol) en solution dans 80 ml de dichlorométhane à 0°C pendant 1 heure.

BrCl est alors ajouté goutte à goutte sur une période de 2 H 55 à la solution de biphényle à 0°C. 15 minutes après la fin de l'addition, on neutralise par une solution aqueuse de sulfite de sodium.

Résultats:

TT                 = 100%
RR (4,4′ diBr)     = 72,2 %
RR (4 - Br)        = 3,0 %

EXEMPLE 5

On suit le même mode opératoire que celui de l'exemple 1 mais en introduisant au départ :
. 0,1 mol biphényle
. 50 ml dichlorométhane
. 0,053 mol de brome
. 0,05 mol de chlore

Le chlore est introduit sous le même débit que dans l'exemple 1 en 26 minutes. Après cette introduction, on neutralise l'excès de brome et on procède comme précédemment.

On récupère une masse de 23,5 g, de composition pondérale suivante :

| . biphényle | 13 % |
| . bromo-4 biphényle | 73,5 % |
| . dibromo-4,4' biphényle | 8,5 % |

| Soit . TT biphényle | 81 % | |
| . bromo-4 biphényle | RR = 74 % | RT = 91,4 % |
| . dibromo-4'4 biphényle | RR = 6 % | RT = 7,4 % |

EXEMPLE 6 comparatif :

On procède comme dans l'exemple 1 mais en ajoutant deux fois plus de brome, à température ambiante et sans ajouter de chlore, on obtient en 21 heures une masse de composition pondérale suivante :
. 37 % de dibromo-4,4′ biphényle
. 57 % de bromo-4 biphényle
. 4 % de bromo-2 biphényle
. 1 % de biphényle

**Revendications**

1. Procédé de bromation du biphényle caractérisé en ce que l'on fait réagir le biphényle avec BrCl.
2. Procédé selon la revendication 1, caractérisé en ce qu'on forme BrCl in situ dans le milieu réactionnel lors de la réaction avec le biphényle.
3. Procédé selon la revendication 1, caractérisé en ce qu'on forme BrCl préalablement à sa réaction avec le biphényle.
4. Procédé selon l'une des revendications précédentes, caractérisé en ce que la réaction a lieu dans un

4

milieu comprenant un solvant.

5. Procédé selon la revendication 4, caractérisé en ce que le solvant est choisi dans le groupe des hydrocarbures aliphatiques, alicycliques, aromatiques halogénés ou non, les éthers, les solvants nitrés.

6. Procédé selon l'une des revendications 1, 2, 4 ou 5 caractérisé en ce qu'on introduit dans le milieu réactionnel le brome dans un premier temps puis le chlore dans un second temps.

7. Procédé selon l'une des revendications 1, 2, 4 ou 5 caractérisé en ce qu'on introduit simultanément dans le milieu réactionnel le brome et le chlore.

8. Procédé selon l'une des revendications précédentes, caractérisé en ce qu'on conduit la réaction avec un excès de brome par rapport au chlore.

9. Procédé selon l'une des revendications précédentes, caractérisé en ce que pendant au moins une partie de la réaction, on refroidit le milieu réactionnel à une température inférieure ou égale à 5°C.

## Claims

1. Process for bromination of biphenyl, characterised in that biphenyl is reacted with BrCl.

2. Process according to Claim 1, characterised in that BrCl is formed in situ in the reaction medium during the reaction with biphenyl.

3. Process according to Claim 1, characterised in that BrCl is formed before its reaction with biphenyl.

4. Process according to one of the preceding claims, characterised in that the reaction takes place in a medium comprising a solvent.

5. Process according to Claim 4, characterised in that the solvent is chosen from the group of halogenated or unhalogenated aliphatic, alicyclic and aromatic hydrocarbons, ethers and nitro solvents.

6. Process according to one of Claims 1, 2, 4 and 5, characterised in that the bromine is introduced into the reaction medium in a first step and then chlorine in a second step.

7. Process according to one of Claims 1, 2, 4 and 5, characterised in that bromine and chlorine are introduced simultaneously into the reaction mixture.

8. Process according to one of the preceding claims, characterised in that the reaction is conducted with an excess of bromine relative to chlorine.

9. Process according to one of the preceding claims, characterised in that the reaction mixture is cooled to a temperature below or equal to 5°C during at least a part of the reaction.

## Patentansprüche

1. Verfahren zum Bromieren von Biphenyl, dadurch gekennzeichnet, daß man Biphenyl mit BrCl umsetzt.

2. Verfahren nach Anspruch 1, dadurch gekennzeichnet, daß man BrCl in situ im Reaktionsmedium während der Reaktion mit Biphenyl bildet.

3. Verfahren nach Anspruch 1, dadurch gekennzeichnet, daß man BrCl vor seiner Reaktion mit Biphenyl bildet.

4. Verfahren nach einem der vorhergehenden Ansprüche, dadurch gekennzeichnet, daß die Reaktion in einem Medium stattfindet, das ein Lösungsmittel enthält.

5. Verfahren nach Anspruch 4, dadurch gekennzeichnet, daß das Lösungsmittel aus der Gruppe der aliphatischen, alizyklischen, halogenierten oder nicht halogenierten aromatischen Kohlenwasserstoffe, der Ether und nitrierten Lösungsmittel ausgewählt ist.

6. Verfahren nach einem der Ansprüche 1, 2, 4 oder 5, dadurch gekennzeichnet, daß man in das Reaktionsgemisch in einer ersten Stufe Brom, dann in einer zweiten Stufe Chlor einbringt.

7. Verfahren nach einem der Ansprüche 1, 2, 4 oder 5, dadurch gekennzeichnet, daß man gleichzeitig Brom und Chlor in das Reaktionsgemisch einbringt.

8. Verfahren nach einem der vorhergehenden Ansprüche, dadurch gekennzeichnet, daß man die Reaktion mit einem Überschuß an Brom bezogen auf Chlor durchführt.

9. Verfahren nach einem der vorhergehenden Ansprüche, dadurch gekennzeichnet, dass man das Reaktionsmedium während mindestens eines Teils der Reaktion auf eine Temperatur unterhalb oder gleich 5°C abkühlt.